(19) Europäisches Patentamt
European Patent Office
Office européen des brevets

(11) Numéro de publication: **0 187 085**
**B1**

(12) # FASCICULE DE BREVET EUROPEEN

(45) Date de publication du fascicule du brevet: **12.09.90**

(51) Int. Cl.$^5$: **C 07 D 401/04,** A 61 K 31/47

(21) Numéro de dépôt: **85402450.2**

(22) Date de dépôt: **10.12.85**

(54) **Dérivés 1-substitués de l'acide 6-fluoro-7-(pyrrol-1-yl)-1,4-dihydro-4-oxoquinoléine-3-carboxylique, leur préparation et leur application en tant que médicaments.**

(30) Priorité: **12.12.84 FR 8419001**

(43) Date de publication de la demande:
**09.07.86 Bulletin 86/28**

(45) Mention de la délivrance du brevet:
**12.09.90 Bulletin 90/37**

(84) Etats contractants désignés:
**AT BE CH DE FR GB IT LI LU NL SE**

(56) Documents cités:
**EP-A- 78 362**
**EP-A-0 134 165**
**EP-A-0 155 244**
**US-A-4 292 317**

**Le dossier contient des informations techniques présentées postérieurement au dépôt de la demande et ne figurant pas dans le présent fascicule.**

(73) Titulaire: **PROVESAN S.A.**
**19, rue de la Croix d'Or**
**CH-1204 Genève (CH)**

(72) Inventeur: **Esteve Soler, José**
**Via Augusta**
**E-244 Barcelone (ES)**

(74) Mandataire: **Ahner, Francis et al**
**CABINET REGIMBEAU, 26, avenue Kléber**
**F-75116 Paris (FR)**

(56) References cited:
**CHEMICAL ABSTRACTS, vol. 102, 1985, page 386, no. 21077v, Columbus, Ohio, US; M. ARTICO et al.: "Reseach on antibacterial and antifungal agents. III. Synthesis of 1-ethyl-1,4-dihydro-4-oxo-7-(1-pyrryl)quinoline-3-carboxylic acid and one of some 6-derivates", & FAMACO, ED. SCI. 184, 39(11), 910-24**
**Wolfson et Hooper, Antimicrobial agents and Chemotherapy, Oct. 1985, 28, No. 4, pp. 581-586**

**EP 0 187 085 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention se rapporte à de nouveaux dérivés 1-substitués de l'acide 6-fluoro-7-(pyrrol-1-yl)-1,4-dihydro-4-oxoquinoléine-3-carboxylique, ainsi qu'à leur préparation et à leur application en tant que médicaments.

Dans la technique antérieure, on connaît déjà des dérivés de l'acide 6-fluoro-7-(pyrrol-1-yl)-1,4-dihydro-4-oxoquinoléine-3-carboxylique présentés comme ayant des propriétés antimicrobiennes. Voir par exemple:

(A) US—4 292 317

(B) Wolfson et Hooper. Minireview: The Fluoroquinolones. Antimicrobial agents and Chemotherapy, Oct. 1985, *28,* No. 4, pages 581—586

(C) EP—A—0 078 362.

La présente invention a eu pour objet la mise au point de nouveaux dérivés de l'acide précité, dotés de propriétés anti bactériennes et fongistatiques améliorées.

Les nouveaux dérivés, objet de la présente invention, répondent à la formule générale I:

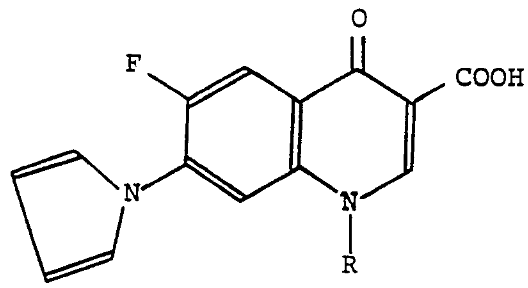

(I)

dans laquelle:

R représente un radical 2-hydroxyéthyle, un radical 2-fluoroéthyle, un radical méthylamino ou un radical éthylamino, ainsi que leurs sels alcalins au alcalinoterreux physiologiquement acceptables.

Les dérivés de formule générale I et leur sels présentent d'intéressantes propriétés pharmacologiques antimicrobiennes en particulier, antibactériennes et fongistatiques.

Les nouveaux composés présentent une puissante activité antibactérienne à la fois à l'égard des bactéries Gram-positives et des bactéries Gram-négatives.

Les nouveaux dérivés de formule générale I peuvent être préparés conformément à l'invention, selon le schéma réactionnel suivant:

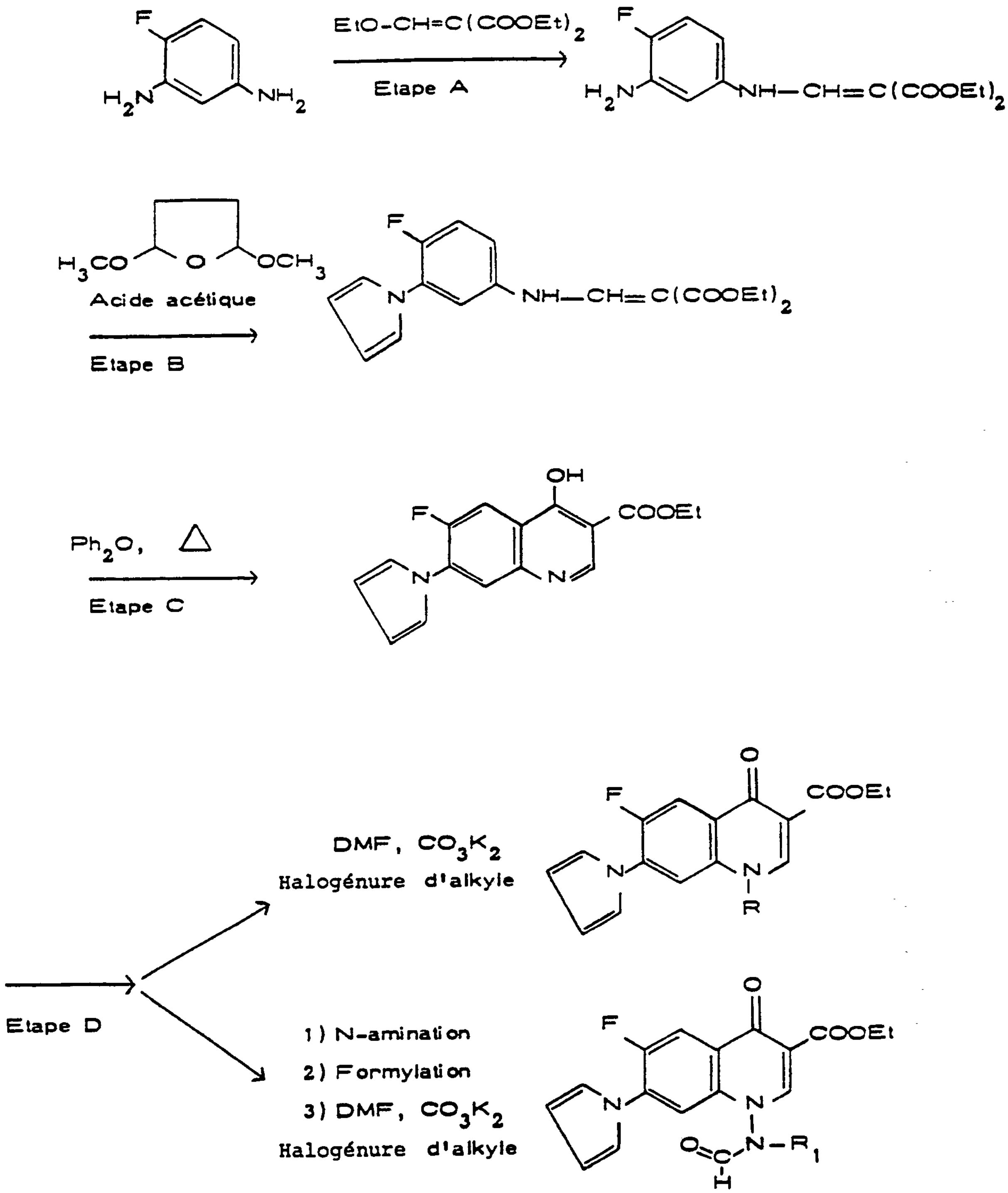


où $R_1$ représente un radical méthyle ou un radical éthyle.

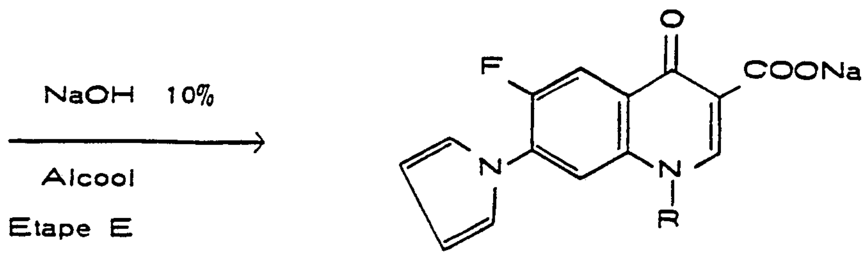


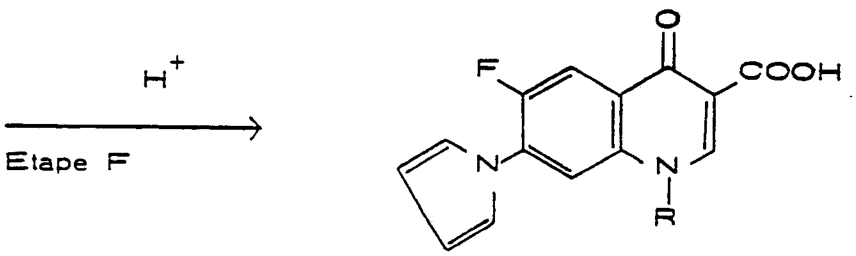


où R a les significations données précédemment.

A l'étape A, la diamine correspondante est condensée directement avec l'éthoxyméthylène malonate de diéthyle pour donner le 3-amino-4-fluor-anilineméthylènemalonate de diéthyle par élimination d'alcool. Au cours de l'étape B, on procède au greffage du noyau pyrrole selon la méthode de Clauson-Kaas, Acta Chem. Scand. *6*, 667 et 867 (1952), par réaction de l'amine et du diméthoxytétrahydrofurane, par reflux en milieu acide acétique. Au cours de l'étape C, le composé est cyclisé pour donner la quinoléine correspondante, par chauffage soit en absence de solvant, soit moyennant l'emploi d'un solvant approprié servant d'échangeur thermique comme par exemple le benzène, le toluène, le xylène, la tétraline, le nitrobenzène, le dichlorobenzène, le diphényléther ou le biphényle, ou encoure un mélange de ces solvants.

La température de réaction est comprise entre 150°C et 250°C, de préférence entre 180—230°C. En employant certains catalyseurs on peut obtenir la cyclisation à des températures beaucoup plus basses. Parmi les catalyseurs appropriés on peut citer à titre d'exemple l'ester polyphosphorique, l'acide polyphosphorique, l'anhydride phosphorique, etc. Avec ces catalyseurs on utilise des températures comprises généralement entre 60—170°C, mieux encore entre 75°C et 150°C.

Puis au cours de l'étape D, on prépare les composés N-alkylés. On peut effectuer l'alkylation en utilisant l'un des agents d'alkylation classiques, lesquels comprennent entre autres, les halogénures d'alkyle, les halogénures d'haloalkyle, les sulfates dialkyliques, les sulfonates d'alkyle, les toluènesulfonates d'alkyle, etc.

En général, la réaction s'effectue en présence d'un alcali et dans un solvant inerte vis-à-vis de la réaction. Les solvants peuvent notamment être constitués par l'eau, le méthanol, l'éthanol, l'acétone, le dioxane, le benzène, le diméthylformamide, le diméthylsulfoxyde, ainsi que des mélanges de ces solvants.

Les alcalis préférés qui peuvent être utilisés sont les hydroxydes alcalins, tels que l'hydroxyde de sodium et l'hydroxyde de potassium, ou bien des carbonates alcalins, tels que le carbonate de sodium ou le carbonate de potassium. Une méthode d'alkylation préférée, qui a lieu en milieu totalement anhydre, s'effectue avec le dimethylformamide comme solvant et le carbonate de potassium comme agent alcalinisant. Les températures de réaction sont généralement comprises entre 60°C et 90°C.

Dans le cas particulier de la préparation de l'acide 6 - fluoro - 7 - (pyrrol - 1 - yl) - 1 - méthylamino - 1,4 - dihydro - 4 - oxo - 3 - quinoléincarboxylique et de l'acide 6 - fluoro - 7 - (pyrrol - 1 - yl) - 1 - éthylamino - 1,4 - dihydro - 4 - oxo - 3 - quinoléincarboxylique, on doit, avant de procéder à l'alkylation, effectuer une N-amination avec un réactif de N-amination tel que par exemple l'acide hydroxylamino-O-sulfonique, le O-mésitylène sulfonylhydroxylamine, le diphénylphosphynyl hdyroxylamine ou le 2,4-dinitrophénylhydroxylamine; puis une activation de l'amine obtenue moyennant procès de formylation. Au cours de l'étape E on effectue une hydrolyse de l'ester par action d'une solution d'hydroxyde de sodium à 10% pendant 3—4 heures à une température de 80—90°C. A l'étape ultime F on acidifie la solution obtenue dans l'étape D avec de l'acide chlorhydrique ou de l'acide acétique pour obtenir les composés objet de la présente invention.

Dans les exemples suivants on indiquera la préparation de nouveaux dérivés selon l'invention ainsi que des matières de départ correspondantes et des produits intermédiaires. On décrira également quelques formes d'emploi typiques pour les différents domaines d'application.

Les exemples ci-après, donnés à simple titre d'illustration, ne doivent cependant en aucune façon limiter l'étendue de l'invention.

Exemple 1

Préparation de 6-fluoro-7-(pyrrol-1-yl)-1-(2-hydroxy-éthyl)-4-oxo-1,4-dihydro-3-quinoléincarboxylate d'éthyle (étape D)

On chauffe pendant 30 minutes à 60°C un mélange de 2 g (0,0076 M) de 6-fluoro-7-(pyrrol-1-yl)-4-hydroxy-3-quinoléincarboxylate d'éthyle et de 1,85 g (2 × 0,0076 M) de carbonate de potassium dans 20 ml de diméthylformamide on laisse refroidir, on ajoute 3,35 g (4 × 0,0076 M) d'éthylenbromhydrine, on chauffe à 80—90°C pendant 10 heures, on ajoute du carbone actif, on filtre à chaud, on évapore à sec, on ajoute de l'eau et on filtre et on lave avec de l'eau le précipité formé. On recristallise avec du diméthylformamide et on obtient 1,2 g d'un solide de point de fusion 237—239°C.

Préparation de l'acide 6-fluoro-7-(pyrrol-1-yl)-1-(2-hydroxyéthyl)-4-oxo-1,4-dihydro-3-quinoléincarboxylique (étapes E et F)

On chauffe à reflux pendant 3 heures un mélange de 5 g de 6-fluoro-7-pyrrol-1-yl)-1-(2-hydroxyéthyl)-4-oxo-1,4-dihydro-3-quinoléincarboxylate d'éthyle, 20 ml d'hydroxyde de sodium à 10%, 20 ml d'éthanol et 35 ml d'eau. On acidifie à chaud avec de l'acide chlorhydrique, on laisse refroidir, on filtre, on lave avec de l'eau, on recristallise avec du diméthylformamide et on obtient 3,6 g d'un solide de point de fusion 230°C.

Données spectroscopiques

$^1H$ RMN, δ, [DMSO($d_6$)]: 3,16 (m, 2H); 4,0 (t, 2H), 4,37 (t, 1H); 5,74 (m, 2H); 6,72 (m, 2H); 7,46 [d($J_{HF}$: 6Hz), 1H]; 7,48 [d($J_{HF}$: 12Hz), 1H]; 8,23 (s, 1H); 14,0 (é, 1H).

IR (KBr): 1620, 1700, 3400 $cm^{-1}$.

Exemple 2

Préparation de 6-fluoro-7-(pyrrol-1-yl)-1-fluoroéthyl-4-oxo-1,4-dihydro-3-quinoléincarboxylate d'éthyle (étape D)

On chauffe pendant 30 minutes à 60°C un mélange de 3,2 g (0,011 M) de 6-fluoro-7-(pyrrol-1-yl)-4-hydroxy-3-quinoléinecarboxylate d'éthyle, 4 g (2,5 × 0,011 M) de carbonate de potassium dans 20 ml de diméthylformamide, on laisse refroidir, on ajoute 7 g (5 × 0,011 M) de bromofluoroéthane, on maintient pendant 7 heures à 80°C, on laisse refroidir, on verse sur un mélange eau/glace, on filtre le précipité formé, on lave avec de l'eau, on recristallise avec un mélange diméthylformamide/eau (1:1) et on obtient 2,35 g d'un solide de point de fusion 231—234°C.

Préparation de l'acide 6-fluoro-7-(pyrrol-1-yl)-1-fluoroéthyl-4-oxo-1,4-dihydro-3-quinoléincarboxylique (étapes E et F)

On maintient à reflux pendant 2,5 heurs un mélange de 0,9 g de 6-fluoro-7-(pyrrol-1-yl)-1-fluoroéthyl-4-oxo-1,4-dihydro-3-quinoléincarboxylate d'éthyle, 15 ml d'eau, 15 ml d'acide chlorhydrique concentré et 15 ml d'éthanol, on laisse refroidir le précipité formé, on filtre, on lave avec de l'eau, on recristallise avec de l'acide acétique et on obtient 0,3 g d'un solide de point de fusion 251—254°C.

Données spectroscopiques

$^1H$ RMN, δ, [DMSO($d_6$)]: 5,0—5,8 (m, 4H), 6,9 (m, 2H); 7,9 (m, 2H); 8,64 [d($J_{HF}$: 6Hz), 1H); 8,71 [d($J_{HF}$: 12Hz); 1H); 9,5 (s, 1H); 13,9 (é, 1H).

IR (KBr): 1620, 1715 $cm^{-1}$.

Exemple 3

Préparation de 6-fluoro-7-(pyrrol-1-yl)-1-amino-4-oxo-1,4-dihydro-3-quinoléincarboxylate d'éthyle (étape D-1)

On chauffe pendant 30 minutes à 60°C un mélange de 6 g (0,02 M) de 6-fluoro-7-(pyrrol-1-yl)-4-hydroxy-3-quinoléincarboxylate d'éthyle et 5,6 g (0,04 M) de carbonate de potassium dans 80 ml de diméthylformamide, on laisse refroidir, on ajoute 8,5 g (0,042 M) de 2,4-dinitrophénylhydroxylamine, on maintient pendant 24 heures à température ambiante, on verse sur 200 ml d'un mélange eau/glace, on filtre le précipité formé, on lave avec de l'eau, on sèche sous dessicateur, on recristallise avec du diméthylformamide et on obtient 1,5 g d'un solide de point de fusion 276—279°C.

Préparation du 6-fluoro-7-(pyrrol-1-yl)-1-formylamino-4-oxo-1,4-dihydro-3-quinoléincarboxylate d'éthyle (étape D-2)

A un mélange de 9,5 ml (0,01 M) d'anhydride acétique et de 4 ml (0,01 M) d'acide formique refroidi à 0°C, on ajoute goutte à goutte de façon à maintenir la température ambiante ±5°C, une solution de 3,15 g (0,01 M) de 6-fluoro-7-(pyrrol-1-yl)-1-amino-4-oxo-1,4-dihydro-3-quinoléincarboxylate d'éthyle dans 25 ml d'acide formique. On maintient à température ambiante pendant 48 heurs, on verse sur un mélange eau/glace, on filtre le précipité formé, on lave avec de l'eau, on recristallise avec un mélange diméthylformamide/eau (1:1) et on obtient un solide de point de fusion 254—257°C.

Préparation du 6-fluoro-7-(pyrrol-1-yl)-formylméthylamino-4-oxo-1,4-dihydro-3-quinoléincarboxylate d'éthyle (étape D-3)

On chauffe pendant 10 minutes à 35°C un mélange de 3,47 g (0,01 M) de 6-fluoro-7-(pyrrol-1-yl)-1-formylamino-4-oxo-1,4-dihydro-3-quinoléincarboxylate d'éthyle et 3 g (~0,02 M) de carbonate de potassium dans 25 ml de diméthylformamide. On maintient à température ambiante pendant 2 heures, partiellement précipité, et on ajoute sur cette suspension 4,3 g (0,03 M) d'iodure de méthyle et on maintient à temperature ambiante pendant 3 heures. On verse sur un mélange eau/glace, on filtre le précipité formé, on lave avec de l'eau, on extrait avec du chloroforme, on filtre, on évapore la solution on recristallise le résidu avec un mélange éthanol/eau (1:1) et on obtient 1,43 g d'un solide de point de fusion 194—198°C.

Préparation de l'acide 6-fluoro-7-(pyrrol-1-yl)-1-méthylamino-4-oxo-1,4-dihydro-3-quinoléincarboxylique (étapes E et F)

On maintient à reflux pendant 2 heures un mélange de 1,43 g de 6-fluoro-7-(pyrrol-1-yl)-1-formylméthylamino-4-oxo-1,4-dihydro-3-quinoléincarboxylate d'éthyle, 8 ml d'hydroxyde de sodium 10%, 40 ml d'eau et 10 ml d'éthanol. On évapore l'éthanol, on acidifie à chaud avec de l'acide acétique, on laisse refroidir, on filtre le précipité, on lave avec de l'eau, on recristallise avec un mélange diméthylformamide/éthanol (1:1) et on obtient 1 g d'un solide de point de fusion 252—256°C.

Données spectroscopiques

$^1$H RMN, δ, [DMSO($d_6$)]: 2,8 (d, 3H); 6,3 (m, 2H); 7,1 (é, 1H); 7,25 (m, 2H); 8,05 [d($J_{HF}$: 6Hz), 1H]; 8,15 [d($J_{HF}$: 11Hz), 1H]; 8,9 (s, 1H); 14,8 (s, 1H).

IR (KBr): 1615, 1710, 3290 $cm^{-1}$.

Exemple 4

Préparation du 6-fluoro-7-(pyrrol-1-yl)-1-formyl-éthylamino-4-oxo-1,4-dihydro-3-quinoléincarboxylate d'éthyle (étape D-3)

On agite pendant 3 heures à température ambiante un mélange de 1,5 g (0,005 M) de 6-fluoro-7-(pyrrol-1-yl)-1-formylamino-4-oxo-1,4-dihydro-3-quinoléincarboxylate d'éthyle et 1,3 g (0,01 M) de carbonate de potassium dans 10 ml de diméthylformamide, puis on ajoute 2,35 g (0,015 M) d'iodure d'éthyle et on maintient pendant 4 heures à température ambiante. On verse sur un mélange eau/glace, on filtre le précipité obtenu, on lave avec de l'eau, on extrait avec du chloroforme, on lave avec de l'eau, on sèche la couche organique, on évapore le solvant, on recristallise le résidu avec un mélange éthanol/eau (1:1) et on obtient 0,7 g d'un solide de point de fusion 192—194°C.

Préparation de l'acide 6-fluoro-7-(pyrrol-1-yl)-1-éthylamino-4-oxo-1,4-dihydro-3-quinoléincarboxylique (étapes E et F)

On maintient à reflux pendant 2 heures un mélange de 0,7 g de 6-fluoro-7-(pyrrol-1-yl)-1-formyléthylamino-4-oxo-4-dihydro-3-quinoléincarboxylate d'éthyle, 5 ml d'hydroxyde de sodium 10%, 25 ml d'eau et 10 ml d'éthanol. On évapore l'éthanol, on acidifie à chaud avec de l'acide acétique, on filtre le précipité formé, on lave avec de l'eau, on recristallise avec un mélange diméthylformamide/éthanol (1:1) et on obtient 0,36 g d'un solide de point de fusion 271—274°C.

Données spectroscopiques

$^1$H RMN, δ, [DMSO($d_6$)]: 1,13 (t, 3H); 3,22 (q, 2H); 6,42 (m, 2H); 7,26 (é, 1H); 7,37 (m, 2H); 8,15 [d($J_{HF}$: 6Hz), 1H]; 8,16 [d($J_{HF}$: 11Hz), 1H]; 9,02 (s, 1H); 14,9 (s, 1H).

IR (KBr): 1620, 1715, 3280 $cm^{-1}$.

*Activité pharmacologique antimicrobienne* (G. L. Daquet et Y. A. Chabbect, Techniques en bactériologie, vol. 3, Flammarion Medecine-Sciences, Paris, 1972 et W. B. Hugo et A. D. Rusell, Pharmaceutical Microbiology, Blackwell Scientific Publications, London, (1977).

*Milieu de culture et solvant:*

Agar d'antibiotiques No. 1 (Oxoid CM 327)
Bouillon de tryptone-soja (Oxoid CM 129)
Solution physiologique Ringer 1/4 (Oxoid BR 52)
Agar Dextrose (BBL-11165)
NaOH 0,1 N

*Microorganismes:*

Bacillus subtilis ATCC 6633
Micrococcus flavus ATCC 10240
Sarcina lutea ATCC 9341
Staphylococcus aureus ATCC 5488/23
Staphylococcus aureus ATCC 25178
Streptococcus faecalis ATCC 10541
Enterobacter aerogenes ATCC 15038
Enterobacter cloacae CHSP-20

Escherichia coli ATCC 10536
Escherichia coli R-1513
Klebsiella pneumoniae ATCC 10031
Citrobacter freundii ATCC 11606
Proteus mirabilis ATCC 4675
Proteus morganii CHSP-16
Pseudomonas aeruginosa 25115
Pseudomonas aeruginosa ADSA 47
Salmonella typhimurium AMES 98
Salmonella typhimurium AMES 100
Serratia marcescens ATCC 13880
Shigella flexnerii

*Préparation des inoculations*

Chacun des microorganismes est ensemencé par strie dans des tubes d'Agar d'antibiotiques No. 1, et mis en incubation pendant 20 heures à 37°C. Ensuite on prend une anse de culture, on ensemence dans un bouillon de Tryptone-soja et on incube pendant 20 heures à 37°C. On dilue à 1/4 la culture obtenue avec une solution physilogique Ringer, de façon à obtenir une suspension normalisée de $10^5$ ufc/ml pour chaque organisme.

*Préparation du milieu contenant les dérivés de formule générale I*

A partir d'une solution de 1000 µg/ml dans NaOH, 0,1 N, chaque produit est dilué dans l'Agar Dextrose (préalablement fondu et maintenu à 50°C) par dilutions successives de façon à obtenir les concentrations suivantes: 64—32—16—8—4—2—1—0,5—0,25—0,125 µg de dérivé/ml du milieu.

Postérieurement, chaque concentrations de chaque produit est répartie dans des boîtes de Petri de 10 cm de diamètre, à raison de 10 ml de milieu par boîte et autant de boîtes que de microorganismes à tester.

Une fois le milieu refroidi, les boîtes sont ensemencées avec les inoculations à raison de 0,4 ml d'inoculation par boîte. On les étend avec une anse de Driglasky et on recuille le surnageant. Les boîtes ensemencées sont incubées à 37°C pendant 20 heures.

*Résultats*

Les résultats obtenus sont décrits dans le tableau I. Les produits des exemples 2, 3, 6 et 7 ont une activité "in vitro" supérieure à celle de l'acide nalidixique, tant à l'égard des Enterobacteriaceas (Pseudomonas aeruginosa) que des coques Gram-positifs. Les dérivés des exemples 1, 4, 5 et 8 présentent une activité du même ordre que l'acide nalidixique face aux microorganismes Gram-négatifs et une activité supérieure face aux coques Gram-positifs. Le dérivé de l'exemple 9 présente une activité supérieure à celle de l'acide nalidixique, à l'égard de tous les microorganismes.

TABLEAU I

CMI "in vitro" comparée à l'acide nalidixique
Les concentrations sont données en µg/ml.

| Microorganismes | Composé de l'exemple 1 | Composé de l'exemple 2 | Composé de l'exemple 3 | Composé de l'exemple 4 | acide nalidixique |
|---|---|---|---|---|---|
| Bacillus subtilis ATCC 6633 | ≤0,062 | ≤0,031 | ≤0,062 | ≤0,062 | 1 |
| Micrococcus flavus ATCC 10240 | 16 | 1 | 4 | 2 | 128 |
| Sarcina lutea ATCC 9341 | 16 | 2 | 4 | 4 | 128 |
| Staphylococcus aureus ATCC 5488/23 | 0,50 | 0,062 | 0,25 | 0,25 | 32 |
| Staphylococcus aureus ATCC 25178 | 0,50 | 0,062 | 0,25 | 0,25 | ≥64 |
| Streptococcus faecalis ATCC 10541 | 1 | 0,50 | 4 | 4 | ≥64 |
| Enterobacter aerogenes ATCC 15038 | 2 | 1 | 1 | 2 | 4 |
| Enterobacter cloacae CHSP-20 | 1 | 0,50 | 1 | 1 | 4 |
| Escherichia coli ATCC 10536 | 0,125 | ≤0,031 | ≤0,062 | 0,062 | 0,25 |
| Escherichia coli R-1513 | 1 | 0,50 | 0,50 | 4 | 4 |
| Klebsiella pneumoniae ATCC 10031 | 0,062 | 0,062 | 0,125 | 0,25 | 1 |
| Citrobacter freundii ATCC 11606 | 1 | 1 | 1 | 4 | 4 |
| Proteus mirabilis ATCC 4675 | 1 | 1 | 2 | 8 | 8 |
| Proteus morganii CHSP-16 | 1 | 0,50 | 1 | 4 | 1 |
| Pseudomonas aeruginosa 25115 | 16 | 4 | 8 | 16 | ≥128 |
| Pseudomonas aeurginosa ADSA 47 | ≥32 | 16 | 16 | ≥32 | ≥128 |
| Salmonella typhinurium AMES 98 | 0,25 | ≤0,031 | 0,125 | 0,062 | 0,50 |
| Salmonella typhinurium AMES 100 | 0,25 | 0,25 | 0,25 | 1 | 0,50 |
| Serratia marcescens ATCC 13880 | 1 | 1 | 1 | 2 | 1 |
| Shigella flexnerii | 1 | 1 | 1 | 1 | 2 |

*Toxicité aiquë chez la souris*

Poue déterminer cette toxicité, on a utilisé comme animaux d'expérimentation des souris albinos de souche C.F.L.P. des deux sexes et d'un poids compris entre 19 et 25 grammes. Après une période de jeûne de 18 heures avec de l'eau "ad libitum", on administre les dérivés objets de la présente invention en suspension dans la gomme arabique à 5% par voie intrapéritonéale. Le volume de la suspension administrée a été dans tous les cas de 0,4 ml/20 grammes (20 ml/kg), en changeant le concentration de la suspension selon la dose administrée.

Une heure après l'administration des dérivés, on fournit aux animaux une nourriture standard rat-souris Panlab. La période d'observation de la mortalité à été de 7 jours. Avec aucun des produits on a observé des différences de mortalité entre les sexes.

Les résultats obtenus sont décrits dans le tableau II.

TABLEAU II

| Dérivés | Voie d'administration | DL-50 mg/kg |
|---|---|---|
| Exemple 1 | i.p. | >1600 |
| Exemple 2 | i.p. | >1600 |
| Exemple 3 | i.p. | >1600 |
| Exemple 4 | i.p. | >1600 |
| Acide Nalidixique | i.p. | 600 |

Compte tenu de leurs bonnes propriétés pharmacologiques, les dérivés de formule générale I sont donc susceptibles d'être utilisés en médecine humaine et/ou vétérinaire, pour le traitement des infections aiguës, chroniques et récidivantes systémiques ou localisées, causées par des microorganismes Gram-positifs et Gram-négatifs sensibles aux produits objet de la présente invention, dans le tractus gastro-intestinal ou génito-urinaire, l'appareil respiratoire, la peau et les tissue mous, ainsi que les infections neurologiques et odonotostomatologiques.

En thérapeutique humaine, la dose proposée des dérivés de la présente invention est environ comprise entre 400 et 1200 mg/jour pour un adulte, par exemple administrée sous la forme de comprimés ou de gélules. Cette posologie peut toutefois varier en fonction de la gravité de l'affection.

On indiquera ci-après, à titre d'exemples, deux formes galéniques particulières des dérivés, objet de la présente invention.

Exemple de formule par comprimé

| | |
|---|---|
| Acide 6-fluoro-7-(pyrrol-1-yl)-1-(2-fluoroéthyl)-1,4-dihydro-4-oxo-3-quinoléincarboxylique | 0,400 g |
| Carboxyméthylamidon | 0,018 g |
| Polyvinylpyrrolidone K29-32 | 0,030 g |
| Cellulose microcristalline | 0,146 g |
| Dioxyde silice colloidale | 0,003 g |
| Stéarate de magnésium | 0,003 g |
| | 0,600 g |

Exemple de formule par gélule

| | |
|---|---|
| Acide 6-fluoro-7-(pyrrol-1-yl)-1-méthylamino-1,4-dihydro-4-oxo-3-quinoléincarboxylique | 0,400 g |
| Cellulose microcristalline | 0,0356 g |
| Dioxyde silice colloidale | 0,0022 g |
| Stéarate de magnésium | 0,0022 g |
| | 0,440 g |

**Revendications**

1. Les dérivés des acides 6-fluoro-7-(pyrrol-1-yl)-1,4-dihydro-4-oxo-quinoléin-3-carboxylique 1-substitués selon la formule générale I

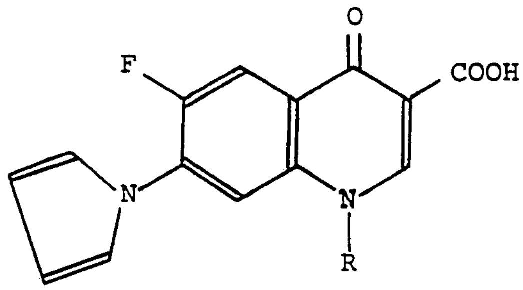

(I)

dans laquelle:

R représente un radical 2-hydroxyéthyle, un radical 2-fluoroéthyle, un radical méthylamino ou un radical éthylamino, ainsi que leurs sels alcalins ou alcalinoterreux physiologiquement acceptables.

2. L'acide 6-fluoro-7-(pyrrol-1-yl)-1-(2-hydroxyéthyl)-1,4-dihydro-4-oxo-quinoléin-3-carboxylique répondant à la formule générale I selon la revendication 1.

3. L'acide 6-fluoro-7-(pyrrol-1-yl)-1-(2-fluoroéthyl)-1,4-dihydro-4-oxo-quinoléin-3-carboxylique répondant à la formule générale I selon la revendication 1.

4. L'acide 6-fluoro-7-(pyrrol-1-yl)-1-méthylamino-1,4-dihydro-4-oxo-quinoléin-3-carboxylique répondant à la formule générale I selon la revendication 1.

5. L'acide 6-fluoro-7-(pyrrol-1-yl)-1-éthylamino-1,4-dihydro-4-oxo-quinoléin-3-carboxylique répondant à la formule générale I selon la revendication 1.

6. Procédé de préparation des dérivés de formule générale I tels que définis dans l'une des revendications 1 à 5, caractérisé par le fait que l'on fait réagir le composé de formule II

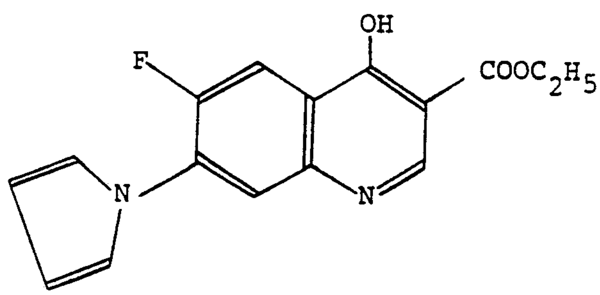

(II)

avec les halogénures d'alkyle correspondant aux significations données à la revendication 1, puis on effectue une hydrolyse alcaline suivie d'une acidification.

7. A titre de médicaments, spécialement comme antibacteriens, les dérivés de formule générale I selon l'une des revendications 1 à 5.

8. Compositions pharmaceutiques, caractérisées par le fait qu'elles contiennent, outre un support pharmaceutiquement acceptable, au moins un dérivé de formule générale I selon l'une des revendications 1 à 5.

**Patentansprüche**

1. Derivate der 6-Fluoro-7-(pyrrol-1-yl)-1,4-dihydro-4-oxo-chinolin-3-carbonsäure, die in 1-Stellung substituiert sind, der folgenden allgemeinen Formel I:

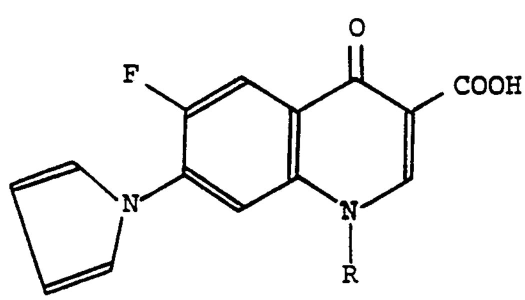

(I)

in der:

R für einen 2-Hydroxyethylrest, einen 2-Fluoroethylrest, einen Methylaminorest oder einen Ethylaminorest steht, einschließlich ihrer physiologisch annehmbaren Alkali- und Erdalkalisalze.

2. 6-Fluoro-7-(pyrrol-1-yl)-1-(2-hydroxyethyl)-1,4-dihydro-4-oxo-chinolin-3-carbonsäure gemäß der allgemeinen Formel von Anspruch 1.

3. 6-Fluoro-7-(pyrrol-1-yl)-1-(2-fluoroethyl)-1,4-dihydro-4-oxo-chinolin-3-carbonsäure gemäß der allgemeinen Formel von Anspruch 1.

4. 6-Fluoro-7-(pyrrol-1-yl)-1-methylamino-1,4-dihydro-4-oxo-chinolin-3-carbonsäure gemäß der allgemeinen Formel von Anspruch 1.

5. 6-Fluoro-7-(pyrrol-1-yl)-1-ethylamino-1,4-dihydro-4-oxo-chinolin-3-carbonsäure gemäß der allgemeinen Formel von Anspruch 1.

6. Verfahren zur Herstellung von Derivaten der allgemeinen Formel I, die in einem der Ansprüche 1 bis 5 definiert sind, dadurch gekennzeichnet, daß man eine Verbindung der Formel II

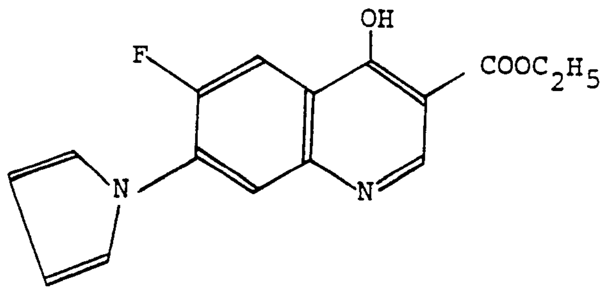

(II)

mit Alkylhalogeniden entsprechend der in Anspruch 1 angegebenen Bedeutung umsetzt, daraufhin eine alkalische Hydrolyse durchführt und anschließend ansäuert.

7. Verwendung der Derivate der allgemeinen Formel I nach einem der Ansprüche 1 bis 5 als Arzneimittel, insbesondere anti-bakterielle Mittel.

8. Pharmazeutische Präparate, dadurch gekennzeichnet, daß sie außer einem pharmazeutisch annehmbaren Träger mindestens ein Derivat der allgemeinen Formel I gemäß einem der Ansprüche 1 bis 5 enthalten.

**Claims**

1. Derivatives of 1-substituted 6-fluoro-7-(pyrrol-1-yl)-1,4-dihydro-4-oxoquinoline-3-carboxylic acids having the general Formula I

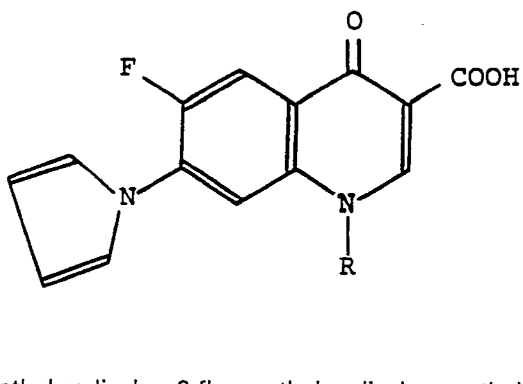

(I)

in which:

R denotes a 2-hydroxyethyl radical, a 2-fluoroethyl radical, a methylamino radical or an ethylamino radical, and their physiologically acceptable alkaline or alkaline earth salts.

2. 6-fluoro-7-(pyrrol-1-yl)-1-(2-hydroxyethyl)-1,4-dihydro-4-oxoquinoline-3-carboxylic acid having the general Formula I according to Claim 1.

3. 6-fluoro-7-(pyrrol-1-yl)-1-(2-fluoroethyl)-1,4-dihydro-4-oxoquinoline-3-carboxylic acid having the general Formula I according to Claim 1.

4. 6-fluoro-7-(pyrrol-1-yl)-1-methylamino-1,4-dihydro-4-oxoquinoline-3-carboxylic acid having the general Formula I according to Claim 1.

5. 6-fluoro-7-(pyrrol-1-yl)-1-ethylamino-1,4-dihydro-4-oxoquinoline-3-carboxylic acid having the general Formula I according to Claim 1.

5. A process for the preparation of derivatives having the general Formula I, as defined in one of Claims 1 to 5, characterized in that the compound of Formula II

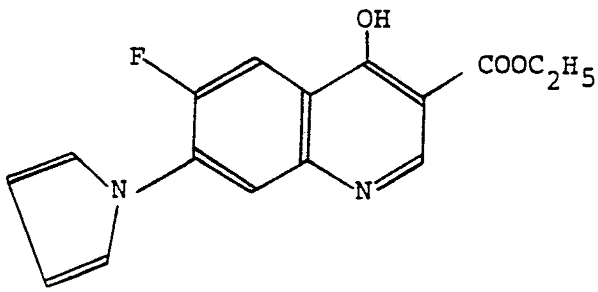

(II)

is reacted with the alkyl halides of the general Formula (I) as defined in Claim 1, whereafter alkaline hydrolysis is effected, followed by an acidification.

7. As medicaments, more particularly antibacterial agents, the derivatives of general Formula I according to any one of Claims 1 to 5.

8. Pharmaceutical compositions, characterized in that they contain, in addition to a pharmaceutically acceptable carrier, at least one derivative of general Formula I according to any one of Claims 1 to 5.